# EUROPEAN PATENT SPECIFICATION

(11) **EP 3 753 580 B1**
(45) Date of publication and mention of the grant of the patent: **13.12.2023**
(21) Application number: 19181697.4
(22) Date of filing: 21.06.2019
(51) Int. Cl.: A61L 2/00, A61L 2/025, B01J 19/00, B01J 19/10, C02F 1/36, B08B 3/12, B08B 7/00, A61L 2/04

(54) **KETTLE DEVICE FOR CLEANING AND STERILIZING SUBMERGED ELEMENTS**
KESSELVORRICHTUNG ZUM REINIGEN UND STERILISIEREN VON UNTERGETAUCHTEN ELEMENTEN
DISPOSITIF DE BOUILLOIRE POUR LE NETTOYAGE ET LA STÉRILISATION D'ÉLÉMENTS IMMERGÉS

(43) Date of publication of application: 23.12.2020
(73) Proprietor: Wibe For Good ApS, 2650 Hvidovre (DK)
(72) Inventor: MØLLER, Kenneth Rueskov, 2720 Vanløse (DK); KJÆR, Morten Hessilt, 2610 Rødovre (DK)
(74) Representative: Andreasen, Søren Laursen Vasegaard

(56) References cited:
- CN-A- 108 746 063
- CN-U- 201 515 946
- CN-U- 204 931 349
- CN-U- 207 152 466
- CN-U- 208 661 935

## Description

### FIELD OF THE INVENTION

The present disclosure relates to an electric kettle comprising an ultrasonic transducer system. More particularly, the disclosure relates to an electric kettle device, to a built-in ultrasonic cleaning system where foreign elements can be cleaned, sterilized and dried within the container of the electric kettle, to a basket containing the foreign elements to be cleaned where the basket can be removed and inserted into the container of the electric kettle, to the heating system of the kettle.

### BACKGROUND OF THE INVENTION

Current ultrasonic cleaning desktop systems use liquid, such as water and added chemical products, to ease the cleaning process, where the added chemical products is often in form of a concentration. The main way of filling the container is by connected water pipes or having a separate container containing water which is supplied to the main container, such as by pouring. Connecting a pipe system to the present-day ultrasonic cleaning system can be problematic in terms of the limited placement options in the container, intervention to piping and time consuming installation.

Not having a connected pipe system requires lifting the entire container system under the water tap, unplugging the system from the power socket before lifting preventing electric shock and reachability issues due to the length of cable being too short. Furthermore most systems will require to be lifted with two hands which is inconvenient. Using a middle step container will require to fill the middle step container, pouring the water into the ultrasonic container with added risk of spilling water.

Current ultrasonic cleaners further contain heating elements for controlling the temperature, ensuring the correct temperature in the liquid for the best cleaning result. These temperatures are not high enough for sterilization of the parts and the main cleaning comes from the added chemicals.

Ultrasonic cleaners are often used with hazardous liquids, such as cleaning agents. This means that parts requires cleaning, if they are to be used in oral or other application coming into contact with food, such as rinsing the parts.

If parts requires cleaning, they are sterilized by a separate cleaning equipment, such as an autoclave or equipment for submerging the equipment in boiling water. These extra cleaning equipment takes up additional space and require handling of several equipment, further complicating the process. A more simple process is therefore desired.

Furthermore, no device induces cavitation with a boiling function, as boiling destroys the small cavitation used for cleaning.

CN207152466U discloses an electrical kettle with ultrasonic cleaning function for primarily outdoor usage, it is useful for cleaning ustensils as it comprises a concave-convex pattern making heating more even and ultrasonic reflection is increased, however this feature increases production cost. Similarly, CN204931349U, CN201515946U and CN108746063A all disclose a kettle comprising a container where the objects to sterilise are placed. All describe an ultrasonic transducer and heater located at the bottom of the container, either adjacent to it, either in a base unit.

Therefore, there is a need to provide a solution that addresses and/or provides an to the above-mentioned problems.

### OBJECT OF THE INVENTION

It may be seen as an object of the present invention to provide a kettle device that solves the above mentioned problems of the prior art.

### SUMMARY OF THE INVENTION

According to an aspect of the invention, a kettle device for cleaning and sterilization of submerged elements is provided. The kettle device comprises
- a kettle unit having a kettle container for receiving liquid in which the elements to be cleaned are submerged during use of the kettle unit, wherein the kettle container (8) is arranged inside the kettle unit (14) wherein a basket (9) with holes for the liquid to flow freely into basket (9) is arranged inside the container (8),
- a base unit for connecting the kettle device to a power supply,
- at least one ultrasonic transducer, positioned at the bottom of the container,
- at least one ultrasonic generator for driving the at least one ultrasonic transducer for generating cavitation in the kettle container, the at least one ultrasonic transducer being with or without direct contact to liquid inside the kettle container, when the kettle device is in use,
- a heating element for heating the liquid during use of the kettle device, and
- a controller for controlling a temperature of liquid in the container (8) during use of the kettle device wherein the heating element is located on the outside of the side portion of the kettle container.

The kettle unit is preferably cordless so that the electric power is transferred thereto via an electric connection between the kettle unit and the base unit.

In some embodiments of the invention, the at least one ultrasonic transducer is fixed to or configured to be fixed to the bottom of the kettle container in the kettle unit. The ultrasonic generator is placed either in the base unit or in the kettle unit. The heating element is configured to be fixed to the kettle container of the kettle unit, on the outside of the side portion of said kettle container (8). unit. The heating element can be controlled to heat the liquid inside the container to a specific temperature in order to sterilize and clean elements submerged in the kettle container. Also the use of cavitation through a liquid media has a sterilizing effect, since the ultrasonic waves above 15 kHz can kill bacteria or spores. The heating element can also work with no liquid present in the kettle container in order to dry the elements already cleaned. The basket is configured to be placed inside the kettle container and can be lifted out from the kettle container.

One disadvantage with the prior art is that the boiling of water destroys the cavitation produced by the ultrasonic generator. A surprising effect was shown in the work leading to the present invention, that by placing the ultrasonic generator in a kettle and controlling the frequency and placement of the elements, it was possible to sustain cavitation small enough to be able to clean and sterilize in the same process. Furthermore, the design may allow for the cavitation of small bobbles, which can clean elements, such as the inside of reusable drinking straws.

In some embodiments of the invention, the kettle unit has a lid adapted to cover an upper opening of the kettle container. Preferably, the lid of the kettle is locked when closed and needs to be released by a release mechanism. E.g. by a press of a button.

The kettle unit may contain at least one handle for manually carrying the kettle unit.

In some embodiments of the invention, the at least one ultrasonic generator is placed in the base unit.

The kettle device comprises an upwardly open basket which is adapted to be placed in and removed from the kettle container, the basked being adapted to contain the elements.

The basket may be made of grid or comprise holes for the ease of cavitation on elements to be cleaned inside the basket and to let elements air dry faster when the basket has been removed from the kettle container. The basket can contain features so that when the basket is placed on a table, these features lift the basket from the underlying surface to let it air dry faster because of increased ventilation from the bottom surface of the basket.

This form of a basket allows the cavitation bobbles to flow unobstructed to the elements to be cleaned, and not burst the bobbles. This may be utilized when the device is used to clean drinking straws, where the cavitation needs to clean the inside of the drinking straws, requiring small cavitation bubbles.

Preferably, a kettle device according to any of the aspects presented herein is provided, wherein the basket comprises stainless steel or titanium. More preferably, the basket is composed of stainless steel.

The basket may be configured to contain locking features to ensure correct placement in the kettle container and attachment-detachment features or additional features to hang the basket to allow cleaned parts to air dry.

The basket may be configured so that the lid of the kettle can be closed while the basket is still placed in the container. Thereby the liquid in the container can be poured out through a spout of the kettle unit without removing elements to be cleaned from the kettle unit prior to emptying water from kettle container.

In some embodiments of the invention, a primary induction coil placed in the base unit uses an iron peg to transfer electric power to a secondary coil placed in the kettle unit to avoid electrical contacts for power supply between the base unit and the kettle unit. This means that the kettle unit and the base unit are easier to clean since no electrical contacts is needed. Furthermore the risk of corrosion on the electrical contact is eliminated since the iron peg and the induction coil are hidden under the enclosure.

Soft material may be placed in part interfaces or sound escaping areas to dampen sound of the at least one ultrasonic transducer.

Preferably, a kettle device according to any of the aspects presented herein is provided, wherein the kettle device comprises one or more sound damping elements in openings between parts of the kettle device and around thin-walled parts to dampen ultrasonic sound escaping from the kettle device. This allows for damping or eliminating the sound, which occurs when the ultrasonic transducer is switched on at hearable frequencies. By adding such damping elements, which is preferably a damping silicone, in openings of the kettle unit, the sound is dampened when the ultrasonic transducer is powered.

In some embodiments of the invention, the kettle unit or the base unit comprises a fan that is used to cool down electronics to avoid overheating, when the kettle device is in use.

In a second aspect, the present invention relates to the use of a kettle device according to the first aspect of the invention for cleaning of one or more of the following:
- professional dental surgical instruments;
- oral care hygiene products, such as toothbrushes, dentures, and braces;
- flatware, such as drinking straws;
- hair dresser accessories, such as scissors, curlers, and coms;
- baby equipment, such as pacifiers, feeding bottles, and teething rings;
- personal sex toys, such as dildos and other genital use toys; and
- kitchen products, such as filters for dishwasher, blenders, and coffee machines.

In one embodiment, the elements may be dried in the kettle device by controlling the temperature of the heating elements, without liquid inside the kettle device.

This allows for the entire cleaning and drying process to be contained within a single device.

These and other aspects of the invention will be apparent from and elucidated with reference to the embodiments described hereinafter.

### BRIEF DESCRIPTION OF THE FIGURES

The aspect of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effects will be apparent from and elucidated with reference to the illustrations described hereinafter in which:
Figure 1 illustrates schematically a cleaning system where the input of 100-250V at 50 or 60Hz can be switched on and off and a controller can adjust an ultrasonic generator frequency which act as input for the ultrasonic transducer and the controller can by use of a temperature sensor control a heating element in the embodiment of the kettle and adjusting the liquid temperature.
Figure 2 illustrates an embodiment of an kettle device, with input parameters of 100-250V at 50 or 60 Hz, connected to a base unit which contains electrical contact points to the kettle enclosure which contains the on/off switch, a controller unit for the ultrasonic generator, heating element, ultrasonic transducer, and temperature sensor.
Figure 3 illustrates schematically a procedure of using the kettle device and the modes of which the kettle device can run.
Figure 4 illustrates a kettle device where induction coils are used to avoid electrical contacts between base unit and kettle unit.
Figure 5 illustrates a kettle device with a built-in fan to avoid overheating of the ultrasonic generator components and transducers within the kettle device.

### DETAILED DESCRIPTION OF AN EMBODIMENT

The detailed description set forth below in connection with the appended drawings is intended as a description of various embodiments of the present invention. The detailed description includes specific details for the purpose of providing a thorough understanding of various concepts. However, it will be apparent to those skilled in the art that these concepts may be practiced without these specific details.

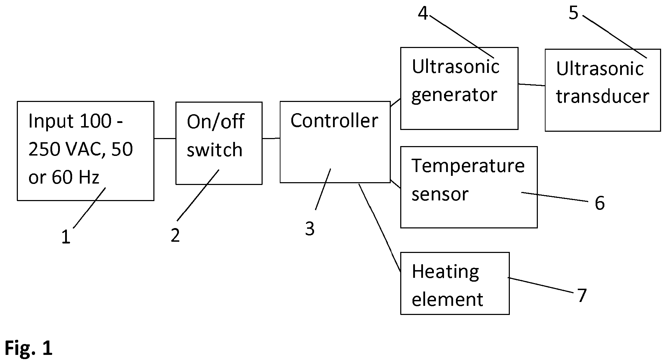

Figure 1 illustrates schematically a cleaning system which will be described in further details below.

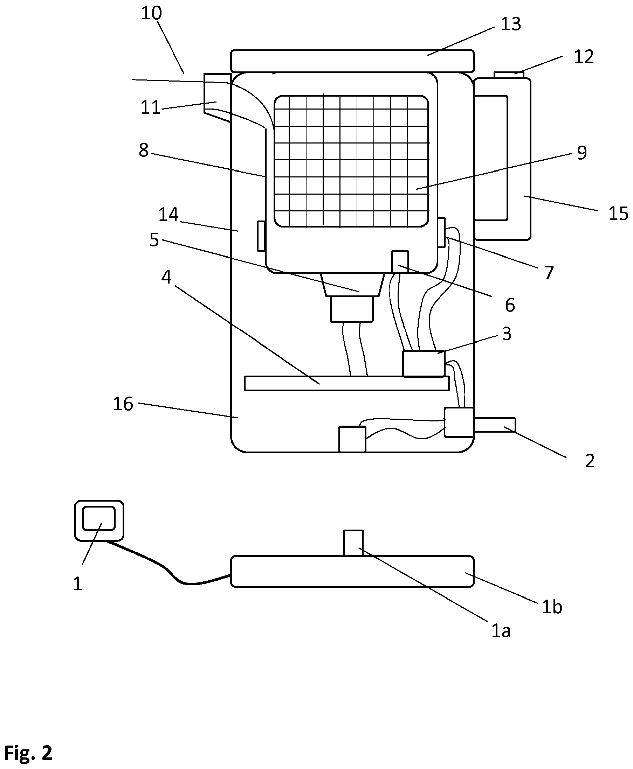

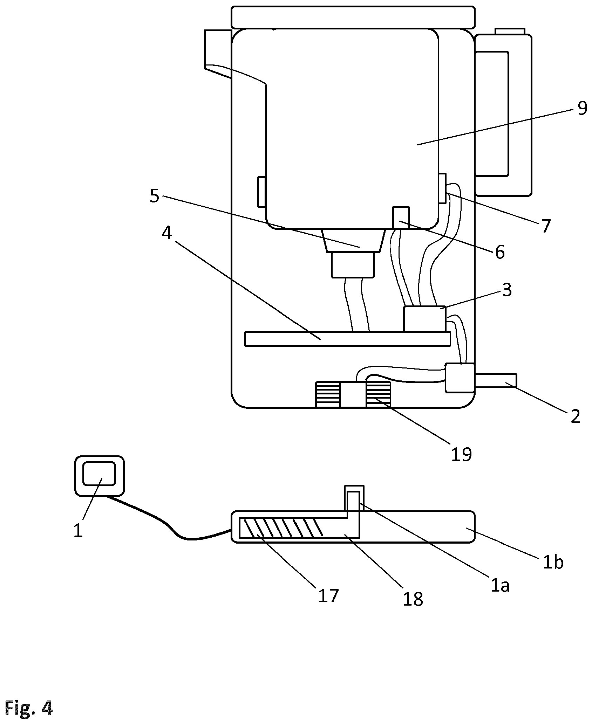

Referring to figure 2 and 4, in one embodiment a kettle device 16 includes a base unit 1b for connecting to power supply 1 which contains a connection adapted to be in electrical contact with the kettle unit 14. The kettle unit 14 contains an on/off switch 2 adapted for switching of the electrically power component of the kettle unit 14. The kettle unit 14 also contains an ultrasonic generator 4 adapted to drive an ultrasonic transducer 5 fixed to the bottom of the container 8 in the kettle unit 14. In the illustrated embodiment, ultrasound generator and the controller is shown as located in the kettle unit 14. However, these parts could also be located in the base unit.

During use of the kettle device 16, the container 8 contains liquid which is energized by the ultrasonic transducer 5. A controller unit 3 controls the frequencies of the ultrasonic transducer 5. The kettle unit 14 contains a heating element 7 fixed to the outside of the side portion of the container 8, which when switched on will heat the liquid inside the container 8. In the illustrated embodiment, attached on the inside of the container 8 is placed a temperature sensor 6 for measuring the temperature of the liquid, where a controller 3 is adapted to control the heating element 7 for adjusting the temperature of the liquid. In preferred embodiments of the invention, the heating element 7 and the ultrasonic generator 4 can be switched on and off independently of each other. Hereby it will e.g. be possible to dry the elements after cleaning without ultrasound being applied.

A meshed basket 9 can be inserted into the liquid, wherein the meshed basket 9 is adapted to contain the parts to be cleaned. The illustrated kettle unit 14 also contains a spout 11 for pouring out water and a lid 13 which can be opened. A release mechanism can open the lid 13 by the press of a button 12, wherein the lid 13 stays closed until the button 12 is pressed. This has the effect that water can be poured from the kettle unit 14 with or without elements in basket 9 when the lid 13 is closed, thereby preventing elements falling out of the container 8.

A kettle device 16 according to the disclosure includes a kettle unit 14 and a base unit 1b. A kettle device 16 may include a kettle unit 14 that is adapted to heat liquid inside a container 8. A heating element 7 may be provided and mounted on the kettle unit container 8.

The kettle unit 14 is adapted to be configured in any known way. This may include corded kettle devices or uncorded kettle devices. It preferably includes uncorded kettle devices.

In general, a kettle device 16 includes i) a base 1b unit connectable to a power supply 1 by an electric plug wherein the base unit 1b contains electric contacts 1a, and ii) a kettle unit 14 containing a container 8 with attached heating element 7 to heat the liquid added to the inside of the container 8 and a switch 2 to stop the heating of the liquid. The kettle device 16 includes at least one ultrasonic transducer 5 coupled to the container 8. The container 8 may include a handle 15, a lid 13 which can be opened by a release mechanism, e.g. a button 12, and a spout 11 where water can be poured. The container 8 may be supplied with temperature sensor 6 measuring the temperature of the liquid inside the container 8, light or display to show on/off state or temperature or wireless connection to wireless electronic devices e.g. using Bluetooth technology where the device output can be controlled or input can be given e.g. time delayed start/stop.

This disclosure further relates to an ultrasonic generator 4 and ultrasonic transducer 5 for cleaning and sterilizing purposes of elements placed in the electric kettle container 8.

The overall idea is to add an ultrasonic transducer 5 to a kettle unit 14 which can clean and sterilize elements placed inside a container 8 by the use of cavitation. Cavitation is the formation of bubbles in a liquid and can be used to remove dirt from the submerged elements.

The elements to be sterilized and cleaned is mounted in a meshed basket 9, the meshed basket 9 is fitted with a basket handle 10 for lifting the basket 9 when the lid 13 of the kettle unit 14 is opened. The base unit 1b contains the electrical cord for power supply 1 with contacts for kettle unit 14. This embodiment makes the handling of the kettle device 16 easier in terms of supplying water from a tap and removing water from the container 8 after sterilization and cleaning.

When cleaned elements have to be removed from the container 8, the basket 9 can be lifted using a basket handle 10 attached to the basket 9 and e.g. place them for drying. Another method is to pour out the liquid in the container 8 and set the kettle device 16 in a drying mode with a low temperature using the heating element 7. By using the temperature sensor 6 and controller 3, the kettle device 16 can go into drying mode based on the input given from the temperature sensor 6.

The base unit 1b contains the electric cord for the power supply 1 and furthermore contains means for electrical transfer to the kettle unit 14. This can either be done by electrical contacts or induction coils 17 and 19. The base unit 1b is adapted to also contain the ultrasonic generator 4. If the ultrasonic generator 4 is placed inside the kettle unit 14 there will be a need of at least two electrical contacts to the base unit 1b if the ultrasonic generator 4 is placed in the base unit 1b four electrical contacts 1a are needed. By using ring contacts, the kettle unit 14 can be placed so as to freely rotate on the base unit 1b which eases handling. The ultrasonic transducer 5 in an embodiment will have a frequency range of approximately 15 kHz to 200 kHz in order to supply sufficient cleaning of submerged elements. The ultrasonic transducer 5 can be fixated on the outside of the container 8 without direct contact to the liquid or inside the container 8 with directly contact with the liquid. It can be placed on the sides of the container 8 or at the bottom of the container 8 or at the lid 13 of the kettle unit 14. The ultrasonic effect of the ultrasonic cleaner corresponds to the container size and distribution. For a container 8 with approximately 0.3 litres to 4 litres the range of the power of the ultrasonic transducer 5 can be from approximately 20 watts to 300 watts. Multiple ultrasonic transducers 5 can ease the distribution of cavitation in the container 8 for parts to be evenly cleaned.

The ultrasonic generator 4 can be made to generate a fixed frequency or sweeping frequency. The sweeping frequency can find the optimum frequency for achieving a resonance and optimize the cavitation effect. The frequency can thereby be optimized even when the amount of liquid varies or when the weight of the part to be cleaned influences the optimum frequency.

The container 8 can be made of stainless steel and have a thickness that allows for cavitation if ultrasonic transducers 5 are mounted on the outside of the container 8. The thickness of the container 8 being approximately 0.1mm to 3mm. The container 8 will have a spout 11 for controlling the pouring of the used liquid and can contain the basket handle 10 when the lid 13 is closed, wherein the basket handle 10 does not collide due to the fact that the closed lid 13 lays in the cavity of the spout 11. The container 8 can a have a volume of approximately 0.3 litres to 4 litres in order allowing the user to lift the kettle unit 14 filled with liquid by using the handle 15 or body of the kettle unit 14.

The basket 9 can be made of stainless steel, plastic, titanium or a combination hereof, preferably stainless steel as it is a cost effective material and furthermore stainless steel is strong which will secure that the basket 9 can have a large open areas and still have a rigid geometry. By having an open structured basket 9, cavitation will occur on the submerged elements and not on the basket 9 surface itself. The basket 9 will have a basket handle 10 which will be shaped so the basket handle 10 can be grabbed by the hand of a user when the basket 9 is mounted in the container 8 of the kettle unit 14. The open structure of the basket 9 can be made of mesh, holes or any other shape. The basket 9 can have cavities for easy separation of the parts submerged or to standardize the placement of elements in the basket 9.

The basket 9 can have features which will make sure that even though the basket 9 is placed on a flat table the basket 9 is lifted, so as to produce a gap between the table and the elements, so as to secure ventilation and faster drying of the cleaned elements. The basket 9 can contain features which will secure alignment in the kettle container 8 to avoid the basket 9 touching the surface of the container 8 as this will influence the effectiveness of the cavitation process and subsequently the cleaning process. This feature can be hooks which align with a recess or protrusion in the container 8, by use of magnets or suitable geometric shape. The basket 9 can also have a basket lid which makes it possible to turn the basket 9 upside down without the cleaned elements falling out. Further, the basket 9 can contain sound damping elements for the spout area if the basket handle 10 is protruding from the kettle unit 14 elsewhere than the spout area.

Now referring to figure 1, which illustrates the schematic of the ultrasonic system in principle where the power supply 1 is connected by a cord to an off/on switch 2 which is connected to a controller 3. The controller is connected to an ultrasonic generator 4 which is connected to at least one ultrasonic transducer 5. The controller 3 is also connected to a temperature sensor 6 which can give input to the controller 3 to adjust the watt usage of a heating element 7.

Figure 2, which illustrates a kettle device 16 according to an embodiment of the disclosure as a perspective view consisting of a base unit 1b and a kettle unit 14.

The kettle unit 14 contains a built-in ultrasonic cleaning system consisting of an ultrasonic generator 4, an ultrasonic transducer 5, a controller 3 which is adapted to control the heating element 7 with input from temperature sensor 6. The base unit 1b has a cord to power supply 1 and electrical contacts to the kettle unit 14 where the kettle unit 14 and base unit 1b is coupled together. The power can be decoupled between the base unit 1b and the kettle unit 14 by an on/off switch 2 which can be operated manually by the user. Inside the kettle unit 14 is a container 8 for containing liquid. Inside the container 8 a basket 9 with holes for the liquid to flow freely into basket and the basket can be inserted and removed by using the basket handle 10 of the basket 9. In the illustrated embodiment, the basket handle 10 lays in the recess of the spout 11 of the kettle container 8. This per se insures that the lid 13 can be closed even though the basket handle 10 is extending out from the kettle unit 14. The spout 11 is further used to pour out the liquid in the container 8. The lid 13 can be closed by pressing down on top of lid 13 with a finger and released by the press of the button 12.

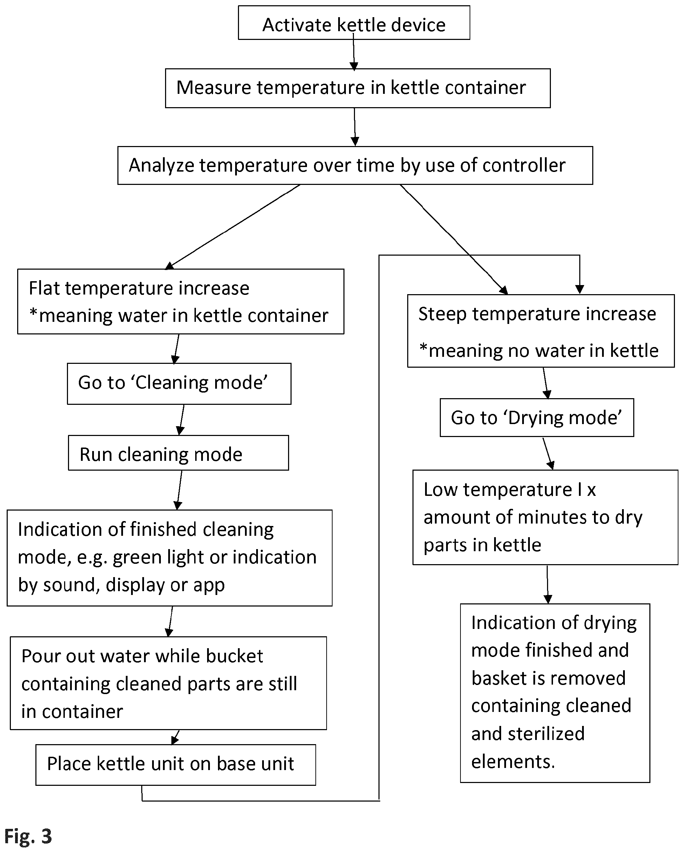

Figure 3 displays a flowchart of a possible operation of the kettle device 16. The kettle device 16 is activated by pressing the on/off switch 2. The controller 3 will read the temperature sensor 6 in the container 8 over a period of time. If the temperature is increasing fast, it means that the container 8 is not containing water. If the temperature is increasing slowly, the container 8 is containing water. If the container is containing water, a cleaning mode will begin. In cleaning mode, both the ultrasonic transducer 5, the temperature sensor 6, and the heating element 7 will be active. When the cleaning program has ended, there will be an indication of finished cleaning mode. The water will now be poured out of the kettle device 16. If the container is not containing water, drying mode will begin. The controller 3 will use the temperature sensor 6 and the heating element 7 to secure a low drying temperature. After a fixed amount of time has passed, the drying mode will end and there will be an indication of finished drying mode.

Figure 4 illustrates a kettle device 16 where induction coils 17 and 19 are used to avoid electrical contacts between the base unit 1b and the kettle unit 14. The primary coil is connected to the power supply 1. The primary coil 17 in the base unit 1b induces a current in the secondary coil 19 in the kettle unit 14 via the iron peg 18 when the kettle unit 14 and the base unit 1b are combined and thereby transfer power from base unit 1b to kettle unit 14.

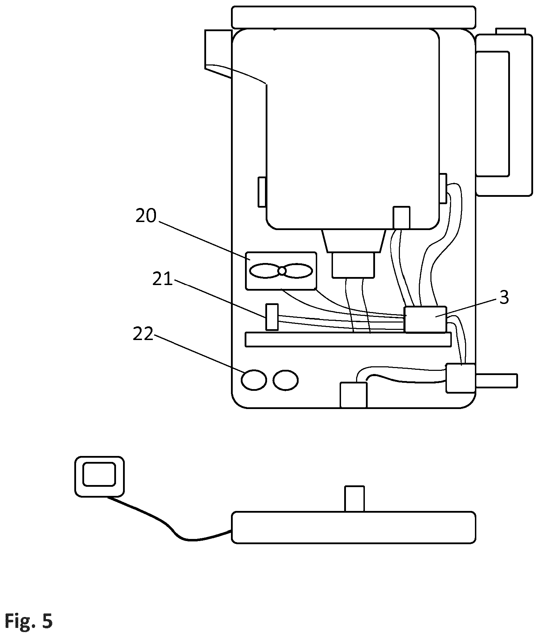

Figure 5 illustrates a kettle device 16 with a built-in fan to avoid overheating of the ultrasonic generator 4 components and the ultrasonic transducers 6 within the kettle device 16. The controller 3 will use input from the fan temperature sensor 21 and thereby adjust the speed of the fan 20 and thereby cool the ultrasonic generator 4 to prevent overheating the system. Holes 22 in the enclosure can be made to allow hot air to be blown out of the enclosure. The fan 20 and the fan temperature sensor 21 can also be added in the base unit 1b if the ultrasonic generator 4 is placed in the base unit 1b.

It is intended that the structural features of the devices described above, either in the detailed description and/or in the claims, may be combined with steps of a method, when appropriately substituted by a corresponding process.

As used, the singular forms "a," "an," and "the" are intended to include the plural forms as well (i.e. to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof. It will also be understood that when an element is referred to as being "connected" or "coupled" to another element, it can be directly connected or coupled to the other element but an intervening elements may also be present, unless expressly stated otherwise. Furthermore, "connected" or "coupled" as used herein may include wirelessly connected or coupled. As used herein, the term "and/or" includes any and all combinations of one or more of the associated listed items. The steps of any disclosed method are not limited to the exact order stated herein, unless expressly stated otherwise.

## Claims

1. Kettle device (16) for cleaning and sterilization of submerged elements, the kettle device (16) comprising:
- a kettle unit (14) having a kettle container (8) for receiving liquid in which the elements to be cleaned are submerged during use of the kettle unit, wherein the kettle container (8) is arranged inside the kettle unit (14), wherein a basket (9) with holes for the liquid to flow freely into basket (9) is arranged inside the container (8);
- a base unit (1b) for connecting the kettle device to a power supply,
- at least one ultrasonic transducer (5), wherein the ultrasonic transducer (5) is positioned at the bottom of the container (8),
- at least one ultrasonic generator (4) for driving the at least one ultrasonic transducer (5) for generating cavitation in the kettle container (8), the at least one ultrasonic transducer (5) being with or without direct contact to liquid inside the kettle container (8), when the kettle device (16) is in use,
- a heating element (7) for heating the liquid during use of the kettle device (16), and
- a controller (3) for controlling a temperature of liquid in the container (8) during use of the kettle device (16), **characterised in that** the heating element (7) is located on the outside of the side portion of the kettle container (8).

2. Kettle device (16) according to claim 1, wherein the kettle unit (14) has a lid (13) adapted to cover an upper opening of the kettle container (8).

3. Kettle device (16) according to any of the preceding claims, wherein the kettle unit (14) contains at least one handle (15) for manually carrying the kettle unit (14).

4. Kettle device (16) according to any of the preceding claims, wherein the at least one ultrasonic generator (4) is placed in the base unit (1b).

5. Kettle device (16) according to any of the preceding claims, wherein said basket (9) is an upwardly open basket (9) which is adapted to be placed in and removed from the kettle container (8), the basked (9) being adapted to contain the elements.

6. Kettle device (16) according to any of the preceding claims, wherein the heating element (7) is formed by the at least one ultrasonic generator (4) so that heat is generated by the vibration of the at least one ultrasonic transducer (5).

7. Kettle device (16) according to any of the preceding claims, wherein a primary induction coil (17) placed in the base unit (1b) uses an iron peg (18) to transfer electric power to a secondary coil (19) placed in the kettle unit (14) to avoid electrical contact between the base unit (1b) and the kettle unit (14).

8. Kettle device (16) according to any of the preceding claims, wherein soft material is placed in part interfaces or sound escaping areas to dampen sound of the at least one ultrasonic transducer (5).

9. Kettle device (16) according to any of the preceding claims, wherein the kettle unit (14) or the base unit (1b) comprises a fan (20) that is used to cool down electronics to avoid overheating, when the kettle device (16) is in use.

10. Use of a kettle device (16) according to any of the preceding claims for cleaning of one or more of the following:
- professional dental surgical instruments;
- oral care hygiene products, such as toothbrushes, dentures, and braces;
- flatware, such as drinking straws;
- hair dresser accessories, such as scissors, curlers, and coms;
- baby equipment, such as pacifiers, feeding bottles, and teething rings;
- personal sex toys, such as dildos and other genital use toys; and
- kitchen products, such as filters for dishwasher, blenders, and coffee machines.

11. Use according to claim 10, wherein the elements are dried in the kettle device by controlling the temperature (3) of the heating elements (7), without liquid inside the kettle device (16).

## Patentansprüche

1. Kesselvorrichtung (16) zur Reinigung und Sterilisation eingetauchter Elemente, wobei die Kesselvorrichtung (16) umfasst:
- eine Kesseleinheit (14) mit einem Kesselbehälter (8) zur Aufnahme von Flüssigkeit, in welche die zu reinigenden Elemente während der Verwendung der Kesseleinheit eingetaucht sind, wobei der Kesselbehälter (8) im Inneren der Kesseleinheit (14) angeordnet ist, wobei ein Korb (9) mit Löchern zum freien Fließen der Flüssigkeit in den Korb (9) im Inneren des Behälters (8) angeordnet ist;
- eine Basiseinheit (Ib) zum Verbinden der Kesselvorrichtung mit einer Stromversorgung,
- zumindest einen Ultraschallwandler (5), wobei der Ultraschallwandler (5) an dem Boden des Behälters (8) positioniert ist,
- zumindest einen Ultraschallerzeuger (4) zum Ansteuern des zumindest einen Ultraschallwandlers (5) zum Erzeugen von Kavitation in dem Kesselbehälter (8), wobei der zumindest eine Ultraschallwandler (5) mit der Flüssigkeit im Inneren des Kesselbehälter (8) direkten Kontakt hat oder nicht, wenn die Kesselvorrichtung (16) in Verwendung ist,
- ein Heizelement (7) zum Erhitzen der Flüssigkeit während der Verwendung der Kesselvorrichtung (16), und
- ein Steuergerät (3) zum Steuern einer Temperatur der Flüssigkeit in dem Behälter (8) während der Verwendung der Kesselvorrichtung (16), **dadurch gekennzeichnet, dass** das Heizelement (7) außerhalb eines Seitenabschnitts des Kesselbehälters (8) befindet.

2. Kesselvorrichtung (16) nach Anspruch 1, wobei die Kesseleinheit (14) einen Deckel (13) aufweist, der dazu geeignet ist, eine obere Öffnung des Kesselbehälters (8) abzudecken.

3. Kesselvorrichtung (16) nach einem der vorhergehenden Ansprüche, wobei die Kesseleinheit (14) zumindest einen Handgriff (15) zum manuellen Tragen der Kesseleinheit (14) enthält.

4. Kesselvorrichtung (16) nach einem der vorhergehenden Ansprüche, wobei der zumindest eine Ultraschallerzeuger (4) in der Basiseinheit (Ib) platziert ist.

5. Kesselvorrichtung (16) nach einem der vorhergehenden Ansprüche, wobei der Korb (9) ein nach oben offener Korb (9) ist, der dazu geeignet ist, in dem Kesselbehälter (8) platziert und daraus entfernt zu werden, wobei der Korb (9) dazu geeignet ist, die Elemente zu enthalten.

6. Kesselvorrichtung (16) nach einem der vorhergehenden Ansprüche, wobei das Heizelement (7) von dem zumindest einen Ultraschallerzeuger (4) gebildet wird, so dass Wärme durch die Schwingung des zumindest einen Ultraschallwandlers (5) erzeugt wird.

7. Kesselvorrichtung (16) nach einem der vorhergehenden Ansprüche, wobei eine primäre Induktionsspule (17) in der Basiseinheit (Ib) einen Eisenstab (18) umfasst, um elektrische Leistung auf eine Sekundärwicklung (19) zu übertragen, die in der Kesseleinheit (14) platziert ist, um elektrischen Kontakt zwischen der Basiseinheit (Ib) und der Kesseleinheit (14) zu vermeiden.

8. Kesselvorrichtung (16) nach einem der vorhergehenden Ansprüche, wobei ein weiches Material in den Schnittstellen zwischen Teilen und schallabstrahlenden Bereichen platziert ist, um den Schall des zumindest einen Ultraschallwandlers (5) zu dämpfen.

9. Kesselvorrichtung (16) nach einem der vorhergehenden Ansprüche, wobei die Kesseleinheit (14) oder die Basiseinheit (Ib) einen Lüfter (20) umfasst, der verwendet wird, um die Elektronik herunterzukühlen, um eine Überhitzung zu vermeiden, wenn die Kesselvorrichtung (16) in Verwendung ist.

10. Verwendung einer Kesselvorrichtung (16) nach einem der vorhergehenden Ansprüche zur Reinigung eines oder mehrerer der folgenden:
- professionale zahnchirurgische Instrumente;
- Mundpflege- und -hygieneprodukte, wie etwa Zahnbürsten, Zahnersatz und Zahnspangen;
- Besteck/Geschirr, wie etwa Trinkhalme;
- Friseurzubehör, wie etwa Scheren, Lockenwickler und Kämme;
- Babyausstattung, wie etwa Schnuller, Trinkfläschchen und Beißringe;
- persönliches Sexspielzeug, wie etwa Dildos und anderes Spielzeug zur genitalen Verwendung; und
- Küchenprodukte, wie etwa Filter für Geschirrspüler, Mixer und Kaffeemaschinen.

11. Verwendung nach Anspruch 10, wobei die Elemente in der Kesselvorrichtung durch Steuern der Temperatur (3) der Heizelemente (7) ohne Flüssigkeit im Inneren des Kesselvorrichtung (16) getrocknet werden.

## Revendications

1. Dispositif de bouilloire (16) pour le nettoyage et la stérilisation d'éléments immergés, le dispositif de bouilloire (16) comprenant :
- une unité de bouilloire (14) ayant un récipient de bouilloire (8) pour recevoir un liquide dans lequel les éléments à nettoyer sont immergés pendant l'utilisation de l'unité de bouilloire, dans lequel le récipient de bouilloire (8) est agencé à l'intérieur de l'unité de bouilloire (14), dans lequel un panier (9) ayant des trous permettant au liquide de s'écouler librement dans le panier (9) est agencé à l'intérieur du récipient (8) ;
- une unité de base (1b) pour relier le dispositif de bouilloire à une alimentation électrique,
- au moins un transducteur ultrasonore (5), dans lequel le transducteur ultrasonore (5) est positionné au fond du récipient (8),
- au moins un générateur d'ultrasons (4) pour entraîner l'au moins un transducteur ultrasonore (5) afin de générer une cavitation dans le récipient de bouilloire (8), l'au moins un transducteur ultrasonore (5) étant avec ou sans contact direct avec le liquide à l'intérieur du récipient de bouilloire (8), lorsque le dispositif de bouilloire (16) est utilisé,
- un élément chauffant (7) pour chauffer le liquide pendant l'utilisation du dispositif de bouilloire (16), et
- un dispositif de commande (3) pour commander une température du liquide dans le récipient (8) pendant l'utilisation du dispositif de bouilloire (16),
**caractérisé en ce que** l'élément chauffant (7) est situé à l'extérieur de la partie latérale du récipient de bouilloire (8).

2. Dispositif de bouilloire (16) selon la revendication 1, dans lequel l'unité de bouilloire (14) a un couvercle (13) adapté pour couvrir une ouverture supérieure du récipient de bouilloire (8).

3. Dispositif de bouilloire (16) selon l'une des revendications précédentes, dans lequel l'unité de bouilloire (14) contient au moins une poignée (15) pour porter manuellement l'unité de bouilloire (14).

4. Dispositif de bouilloire (16) selon l'une des revendications précédentes, dans lequel l'au moins un générateur d'ultrasons (4) est placé dans l'unité de base (1b).

5. Dispositif de bouilloire (16) selon l'une des revendications précédentes, dans lequel ledit panier (9) est un panier ouvert vers le haut (9) qui est adapté pour être placé dans et retiré du récipient de bouilloire (8), le panier (9) étant adapté pour contenir les éléments.

6. Dispositif de bouilloire (16) selon l'une des revendications précédentes, dans lequel l'élément chauffant (7) est formé par l'au moins un générateur d'ultrasons (4) de sorte que de la chaleur soit générée par la vibration de l'au moins un transducteur ultrasonore (5).

7. Dispositif de bouilloire (16) selon l'une des revendications précédentes, dans lequel une bobine d'induction primaire (17) placée dans l'unité de base (1b) utilise une cheville en fer (18) pour transférer de l'énergie électrique à une bobine secondaire (19) placée dans l'unité de bouilloire (14) pour éviter un contact électrique entre l'unité de base (1b) et l'unité de bouilloire (14).

8. Dispositif de bouilloire (16) selon l'une des revendications précédentes, dans lequel un matériau souple est placé dans des interfaces entre pièces ou des zones d'échappement de son pour atténuer le son de l'au moins un transducteur ultrasonore (5).

9. Dispositif de bouilloire (16) selon l'une des revendications précédentes, dans lequel l'unité de bouilloire (14) ou l'unité de base (1b) comprend un ventilateur (20) qui est utilisé pour refroidir les composants électroniques afin d'éviter la surchauffe, lorsque le dispositif de bouilloire (16) est utilisé.

10. Utilisation d'un dispositif de bouilloire (16) selon l'une des revendications précédentes pour le nettoyage d'un ou de plusieurs de ce qui suit :
- des instruments de chirurgie dentaire professionnels ;
- des produits d'hygiène bucco-dentaire, tels que des brosses à dents, des prothèses dentaires et des appareils dentaires ;
- des couverts, tels que des pailles ;
- des accessoires de coiffure, tels que des ciseaux, des bigoudis et des peignes ;
- du matériel pour bébé, tel que des tétines, des biberons et des anneaux de dentition ;
- des jouets sexuels personnels, tels que des godemichés et autres jouets à usage génital ; et
- des produits de cuisine, tels que des filtres pour lave-vaisselle, mixeurs et machines à café.

11. Utilisation selon la revendication 10, dans laquelle les éléments sont séchés dans le dispositif de bouilloire en commandant la température (3) des éléments chauffants (7), sans liquide à l'intérieur du dispositif de bouilloire (16).
